# EUROPEAN PATENT SPECIFICATION

(11) **EP 2 709 712 B1**
(45) Date of publication and mention of the grant of the patent: **17.04.2019**
(21) Application number: 12784868.7
(22) Date of filing: 16.05.2012
(51) Int. Cl.: A61M 29/02, A61B 17/00, A61B 5/0215, A61M 25/06, A61M 25/00, A61M 25/01, A61M 29/00, A61B 5/026, A61B 90/00

(54) **SHEATH-DILATOR SYSTEM**
HÜLLENDILATATORSYSTEM
SYSTÈME DE DILATATION DE GAINE

(30) Priority: 16.05.2011 US 201161486637 P
(43) Date of publication of application: 26.03.2014
(73) Proprietor: Vivasure Medical Limited, Galway H91 V3KP (IE)
(72) Inventor: MARTIN, Christopher, Oughterard County Galway (IE); RYAN, Damien, Headford Road Galway (IE); BRETT, Gerard, Claregalway County Galway (IE)
(74) Representative: O'Brien, John Augustine
(86) International application number: PCT/IB2012/001101
(87) International publication number: WO 2012/156819

(56) References cited:
- WO-A2-02/102236
- US-A- 5 676 689
- US-A1- 2002 107 506
- US-A1- 2002 169 377
- US-A1- 2004 082 906
- US-A1- 2004 082 906
- US-A1- 2009 088 723
- US-A1- 2009 312 786
- US-B1- 6 485 481

## Description

### CROSS-REFERENCE TO RELATED APPLICATIONS

This application claims the benefit of priority to U.S. Provisional Patent Application Serial No. 61/486,637, filed May 16, 2011.

### TECHNICAL FIELD

The present invention relates generally to a sheath-dilator system for use in surgical procedures.

### BACKGROUND

In endoscopic and endovascular surgery, when access to hollow vessels is required via a conduit suitable for transporting or engaging therapeutic equipment and materials, introducer sheath-dilators are routinely used. Typically in endovascular surgery, the dilator and the introducer sheath have radiopaque characteristics to aid in visualization of these items within the body. Additionally, the introducer sheath may use radiopaque markers on the tip. These markers provide an indicator of tip location in the body when viewed fluoroscopically. The radiopaque tip is commonly comprised of a metal band, made of a radiopaque material such as gold on the distal tip of the introducer sheath. Such materials may substantially increase the cost associated with the production of such devices and may provide undesirable geometric constraints that create obstacles in connection with delivering therapeutic equipment through the introducer sheath, particularly when such equipment or materials are non uniform in size and shape.
US5,676,689 describes a haemostatic puncture closure system according to the preamble of claim 1.
US20020169377A describes a method and apparatus for accessing the left atrical appendage. The apparatus may comprise a dilator with a distal end with a signal transmitting surface and a signal receiving surface for transmission of, for example, visible light.
US20040082906A describes a vascular suction cannula, dilator and surgical stapler.

### SUMMARY

The present invention provides a sheath dilator system. In some embodiments, a sheath dilator includes a blood detector, which may be used to calibrate the location of the sheath-dilator system with respect to an artery.

The invention is defined in claim 1.

The medical device includes: a sheath having a proximal end and a distal end, the sheath having an opening at both the proximal end and the distal end; and a removable dilator having a distal end and a proximal end, the dilator having at least one blood detector disposed on a tapered portion of the distal end of the dilator, the dilator positionable within the sheath such that the tapered portion of the distal end of the dilator extends through the opening in the distal end of the sheath, the proximal end of the dilator extends through the opening in the proximal end of the sheath, and at least a portion of the at least one blood detector is adjacent to the distal end of the sheath.

The dilator includes a blood inlet opening disposed on the tapered portion. The blood inlet opening may have a crescent-shaped cross-section when viewed along a longitudinal axis of the dilator.

The sheath may include one or more depth indicators affixed thereto.

The at least one blood detector may include a blood inlet opening in fluid communication with a blood outlet at the proximal end of the dilator.

The at least one blood detector may be a pressure sensor.

The at least one blood detector may be a flow sensor.

The at least one blood detector may be a tissue type sensor.

The sheath may include a hub at the proximal end of the sheath, and the dilator may include a hub at the proximal end of the dilator, the sheath hub releasably engaged with the dilator hub.

The blood outlet may be in the dilator hub.

The dilator may include at least one opening at the distal tapered end, the at least one opening in fluid communication with one or more lumens within the dilator, the one or more lumens in fluid communication with a port in the dilator hub, whereby liquid is transferable between the opening in the distal tapered end of the dilator and the port in the dilator hub.

The at least one sensor may be connected to a control system and a display unit.

The depth indicators may include graduation marks.

The medical device may further include a luer connector extending from the port in the dilator hub, the luer connector creating a fluid pathway between the port and at least one of a 2 and 3 way tap.

The dilator may include an internal lumen extending the entire length of the dilator and adapted for tracking over a guide wire.

The transition between the distal end of the sheath and the dilator may be substantially smooth.

The sheath hub may include an interface connectable to an endo vascular device.

The endovascular device may be a closure device.

The medical device may further include at least one sensor on the outer surface of the sheath.

The medical device may include a linear array of sensors located along the outer surface of the sheath.

The linear array of sensors may be an array of tissue type sensors.

The at least one sensor may include a pressure sensor.

The medical device may include one or more radiopaque markers positioned on the dilator.

Also described is a medical device which includes: a sheath having a proximal end and a distal end, the sheath having an opening at both the proximal end and the distal end; and a removable dilator having a distal end and a proximal end, the dilator being positionable within the sheath such that the tapered portion of the distal end of the dilator extends through the opening in the distal end of the sheath, the proximal end of the dilator extends through the opening in the proximal end of the sheath, wherein the dilator further includes a plurality of flushing ports disposed adjacent the distal end of the sheath when the dilator is positioned within the sheath, and a plurality of detector ports, separate from the flushing ports, being disposed on a tapered portion of the distal end of the dilator.

At least one of the detector ports may have an elliptical cross-section when viewed along a longitudinal axis of the dilator.

At least one of the detector ports may have a crescent-shaped cross-section when viewed along a longitudinal axis of the dilator.

Also described is a method of positioning a medical device which includes: inserting a distal end of a dilator releasably engaged with a sheath having depth indicators thereon into an artery, the dilator including at least one blood detector positioned on a tapered portion of the distal end of the dilator, the distal end of the dilator extending through an opening in the distal end of the sheath; monitoring the blood detector for a signal indicating entry of the blood detector into the artery contemporaneously with inserting the dilator into the artery; and referencing the position of the sheath with respect to the artery based on the location of the sheath with respect to the patient upon generation of the signal indicating entry of the blood detector into the artery.

The blood detector may include a blood inlet opening disposed on the tapered portion, the blood inlet opening having an elliptical cross-section when viewed along a longitudinal axis of the dilator.

The blood detector may include a blood inlet opening disposed on the tapered portion, the blood inlet opening having a crescent-shaped cross-section when viewed along a longitudinal axis of the dilator.

The at least one blood detector may be a blood inlet opening in fluid communication with a blood outlet at the proximal end of the dilator.

The sheath may include a hub at the proximal end of the sheath and the dilator comprises a hub at the proximal end of the dilator, the dilator releasably engaged with the sheath via a connection between the dilator hub and the sheath hub.

Also described is a method of positioning a medical device which includes: inserting a distal end of a dilator releasably engaged with a sheath having depth indicators thereon into an artery, the dilator including at least one blood detector positioned on the distal end of the dilator, the distal end of the dilator extending through an opening in the distal end of the sheath, wherein the dilator further includes a plurality of flushing ports disposed adjacent the distal end of the sheath when the dilator is engaged with the sheath, and a plurality of detector ports, separate from the flushing ports, being disposed on a tapered portion of the distal end of the dilator; monitoring the blood detector for a signal indicating entry of the blood detector into the artery contemporaneously with inserting the dilator into the artery; and referencing the position of the sheath with respect to the artery based on the
location of the sheath with respect to the patient upon generation of the signal indicating entry of the blood detector into the artery.

At least one of the detector ports may include a blood inlet opening disposed on the tapered portion, the blood inlet opening having an elliptical cross-section when viewed along a longitudinal axis of the dilator.

At least one of the blood detectors may include a blood inlet opening disposed on the tapered portion, the blood inlet opening having a crescent-shaped cross-section when viewed along a longitudinal axis of the dilator.

The method may further include flushing a saline solution through the flushing ports.

At least one blood detector may include a blood inlet opening in fluid communication with a blood outlet at the proximal end of the dilator.

The sheath may include a hub at the proximal end of the sheath and the dilator may include a hub at the proximal end of the dilator, the dilator releasably engaged with the sheath via a connection between the dilator hub and the sheath hub.

Further features and aspects of example embodiments are described in more detail below with reference to the appended Figures. Details of the sheath dilator system, and uses thereof, are described herein, *infra.*

### BRIEF DESCRIPTION OF THE DRAWINGS

FIG. 1 is a side view of a sheath-dilator assembly tracked over a guide wire, in accordance with embodiments.
FIG. 2 is a side view of the sheath-dilator of FIG. 1 advanced into an artery.
FIG. 3 is a side view of the sheath-dilator of FIGS. 1 and 2 advanced further into the artery.
FIG. 4 is a side view of the sheath-dilator of FIGS. 1-3 post withdrawal of the dilator component from the system, in accordance with embodiments.
FIG. 5 shows a schematic of a dilator hub with a blood signal port connected to a syringe in accordance with embodiments.
FIG. 6 is an isometric view showing a blood inlet hole behind tapered tip of dilator, in accordance with embodiments.
FIG. 7 illustrates cross-sectional end views of various dilators having distinct blood inlet hole configurations in accordance with embodiments.
FIG. 8 is an isometric and front view of a sheath-dilator having an elliptical blood inlet hole on the tapered section of the dilator portion in accordance with embodiments.
FIG. 9 is a cross section of a dilator with a multi-lumen configuration in accordance with embodiments.
FIG. 10 is a side view and a cross sectional view of a multi-lumen dilator having flushing holes in accordance with embodiments.
FIG. 11 illustrates a sheath-dilator having blood pressure sensors in accordance with embodiments.
FIG. 12 shows a sheath assembly having sensors connected to a control unit and a display in accordance with embodiments.
FIG. 13 shows an isometric view of a sheath having embedded sensors in accordance with embodiments.
FIG 14 illustrates a hub for a dilator in accordance with embodiments.
FIG 15 illustrates another hub for a sheath-dilator system in accordance with embodiments.
FIG. 16 shows a sheath dilator system that includes radiopaque markers in accordance with embodiments.
FIG. 17 shows a sheath dilator system that includes radiopaque markers in accordance with embodiments.

### DETAILED DESCRIPTION OF CERTAIN EMBODIMENTS

### 1. General Description of Certain Embodiments :

As described herein, the present invention provides a sheath-dilator system (also referred to herein as a "device"). Such, a device is useful for insertion and removal of fluids and introduction of medical devices and systems, such as devices and systems used in arterial closure operations.

Embodiments are specially adapted to provide an operator with an indication of the position of the introducer sheath with respect to a vessel. Once the operator knows the location of the tip of the sheath, the operator may advance the sheath-dilator assembly into the vessel accurately at fixed distance inside a vessel. Embodiments provide various mechanisms for indicating when the introducer sheath enters a vessel and for monitoring the penetration depth subsequent to that entry, including in some embodiments a passive detection system.

In accordance with embodiments, a medical device is provided that includes a sheath and a dilator. The sheath has a proximal end and a distal end each of which include an opening. The sheath also includes one or more depth indicators affixed thereto. The dilator is removably positioned within the sheath and includes a distal tapered end, a proximal end, and at least one blood detector. The dilator is positioned within the sheath such that the distal tapered end extends through the opening in the distal end of the sheath and the proximal end of the dilator extends through the opening in the proximal end of the sheath. At least a portion of the at least one blood detector on the dilator is adjacent to the distal end of the sheath.

Some examples provide a method of positioning a medical device that includes a sheath and a dilator. Examples according to those methods require inserting a distal tapered end of a dilator releasably engaged with a sheath having depth indicators thereon into an artery. The dilator includes at least one blood detector positioned on the distal end of the dilator and extending through an opening in the distal end of the sheath. The method further requires monitoring the blood detector for a signal indicating entry of the blood detector into the artery contemporaneously with inserting the dilator into the artery and requires referencing the position of the sheath distal tip with respect to the artery based on the location of the sheath with respect to the patient upon generation of the signal indicating entry of the blood detector into the artery.

### 2. Aspects Embodied by the Figures:

Other aspects, features and advantages of the presently disclosed sheath-dilator system will become apparent from the following detailed description taken in conjunction with the accompanying drawing, which illustrate, by way of example, embodiments of the presently disclosed systems and methods.

Referring now to the drawing figures, wherein like references numerals identify similar, identical or corresponding elements, embodiments of the presently disclosed sheath-dilator system are described.

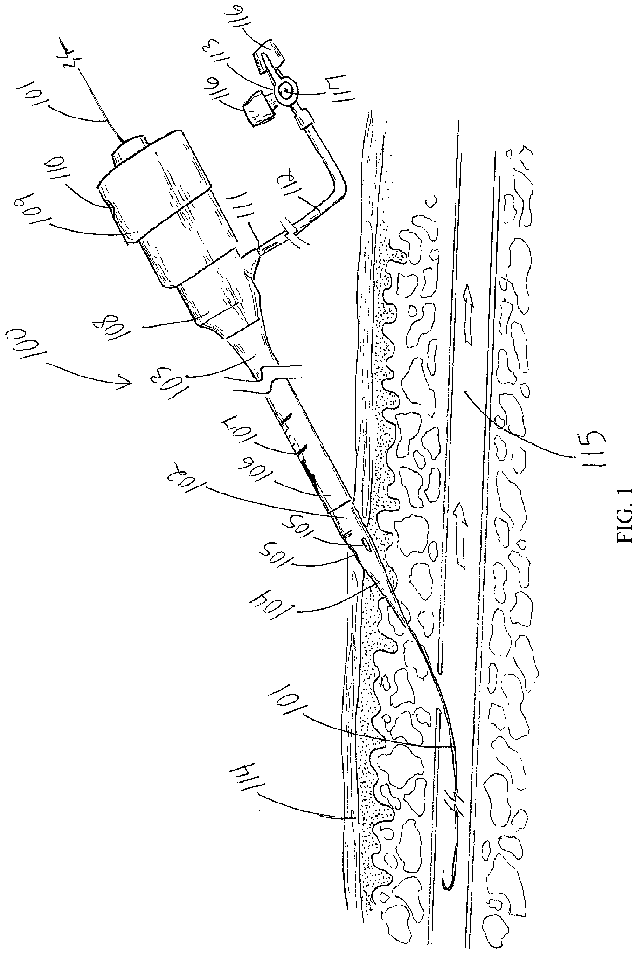

As demonstrated in FIGS. 1-4 embodiments provide a system for positioning the sheath tip a fixed distance within a blood vessel through a blood signaling system on the dilator component. The sheath-dilator 100 is inserted through a patient's skin 114 and the underlying tissue layers to reach a vessel 115 such as a femoral artery. The system may be inserted specifically such that the angle of insertion is composed of a lateral component that opposes the direction of flow of the blood within the vessel 115 as demonstrated in FIG. 1. As further described below, doing so will allow the operator to know the exact position of the sheath tip in relation to the arteriotomy in the vessel wall, thereby allowing the operator to advance the sheath tip a specific, required distance, for example 20 mm, inside the artery by referencing depth indicators 107, which may be graduated markings on the outer surface of the sheath. The graduated markings on the sheath will be referenced against the skin surface when the initial blood signal is observed.

With this reference point fixed, the user will then be able to advance the sheath the required distance inside the artery by advancing the graduated makings this distance against the same reference point on the skin surface.

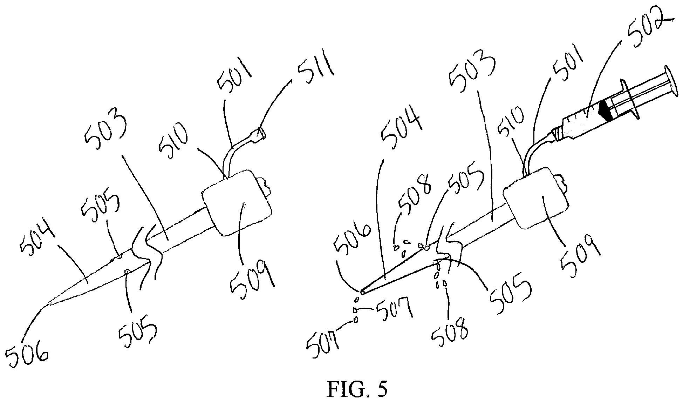

FIG. 1 provides a side view of the initial stage in the process described above. FIG. 1 demonstrates a cross-sectional side view of vessel 115 and a side view of sheath-dilator 100 entering the patient's skin 114. The sheath-dilator system 100 is tracked over a guide wire 101 that is left in place once an introducer sheath used for the main interventional procedure is removed from the femoral artery 115, in accordance with embodiments. The lumen in the dilator through which the guide wire travels fits guide wire 101 at distal tip portion 104 via a hole (see holes 506, 606, and 806 at the dilator tips shown in Figs. 5, 6, and 8 respectively) such that the dilator freely moves on the wire, yet minimizes blood leakage around the wire into the lumen through which the wire extends. In accordance with some embodiments, following removal of the initial introducer sheath and prior to insertion of sheath-dilator 100, manual pressure is applied to the wound site to minimize blood loss during this sheath exchange. Subsequently, sheath-dilator 100 is tracked over guide wire 101 and down through the tissue tract towards the artery 115. It should be understood however, that the sheath dilator system 100 may be used without sheath exchange in accordance with example embodiments.

Sheath-dilator 100 includes a dilator 102 removably positioned within sheath 103. The distal tapered tip 104 of dilator 102 extends through an opening in the distal end 106 of sheath 103. The dilator assists in the delivery of the introducer sheath by the most atraumatic means. The dilator includes blood inlet holes 105, which help detect blood flow and hence entry into artery 115. The blood inlet holes 105 are particularly suitable for blood detection in view of their location on the tapered section of dilator 102. As discussed herein the blood inlet holes may be used for the insertion of a contrast agent, the detection of which may also be used to indicate dilator and sheath location. The sheath contains graduation marks 107, which help indicate and track the insertion depth of the sheath into the artery once an entry reference point is established as described above and as further discussed below. The graduation marks 107 indicated and track insertion position accurately when the sheath 103 and the dilator 102 are held connected to each other in a fixed relative position, such that the sheath 103 does not move relative to the dilator 102 during insertion. The embodiment demonstrated in FIG. 1 also includes a hub 108 on the proximal end of sheath 103. Hub 108 includes an opening through which dilator 102 is inserted. Dilator 102 also includes hub 109 at the proximal end of dilator 102. Hub 109 is releasably engageable with hub 108 in accordance with various embodiments. The releasable engagement may be achieved through various mechanisms such as a twist and lock system, external latches, threaded engagement, or other connection systems. This embodiment also demonstrates blood outlet opening 110 located on hub 109. Blood outlet 110 signals blood flow emanating from entry of blood into inlet 105 after inlet 105 passes into the interior region of femoral artery 115.

The embodiment depicted in FIG. 1 further depicts a port 111 located on hub 108. Tube 112 connects port 111 to luer connector 113. Luer connector 113 provides two connection sites 116. The luer connector further includes a valve 117 for controlling flow between systems connected at the connection sites 116 to port 110 through tube 112 and connector 113. Accordingly, connector 113 provides various insertion and extraction fluid channels.

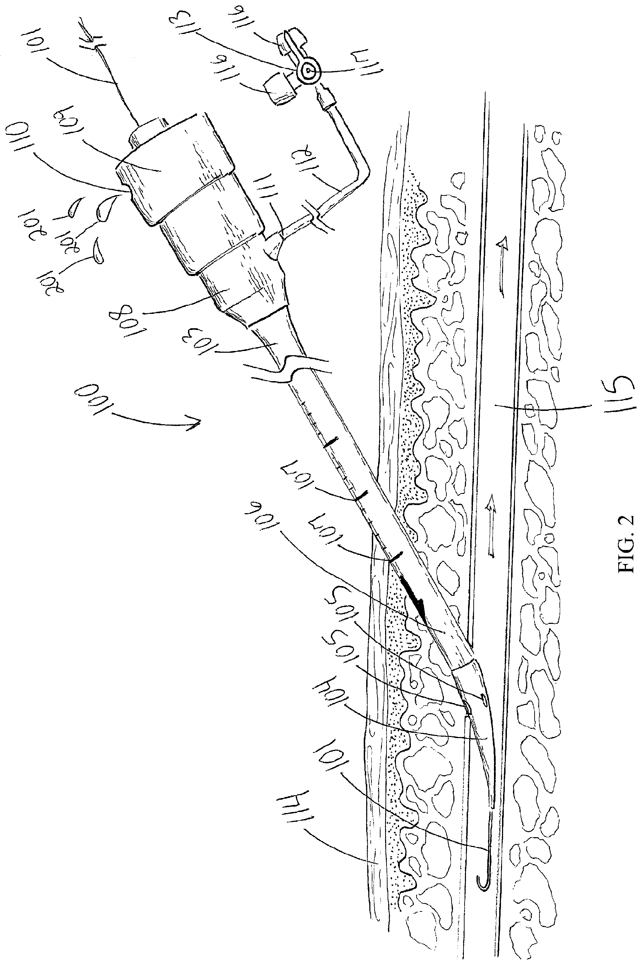

FIG. 2 provides a side view of the sheath-dilator of FIG. 1 advanced into femoral artery 115 and advanced further along guide wire 101. As demonstrated in FIG. 2, an operator advance the distal tapered tip of the dilator through the arteriotomy in artery 115 until the blood inlet holes 105 are inside of the artery. The operator is apprised of the entry of blood inlet holes 105 into the artery because entry of the holes in the artery initiates an arterial pulsatile flow of blood through the inlet holes 105 into one or more channels within the dilator and out of blood outlet 110. Blood 201 flowing out of outlet 110 confirms entry of the inlet holes 105 into artery 115. Furthermore, because the blood inlet holes are adjacent to the distal tip 105 of sheath 103, the blood indicator signals the entry of the sheath into the artery. Once the operator knows that the sheath has entered or is substantially at the point of entry into the artery 115, the operator may reference a specific mark 107 on the sheath 103 adjacent to the patient's skin 114. Accordingly, the blood flow effectively calibrates the indicators 107.

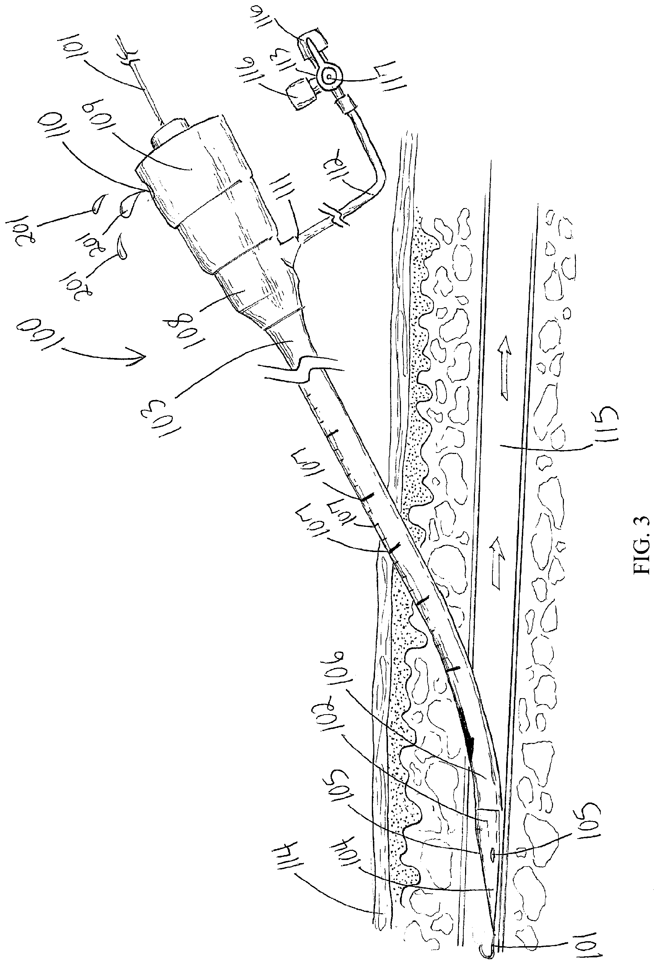

As shown in FIG. 3 the operator may continue advancing the sheath dilator 100 down the guide wire and further into the artery and the depth of penetration will be indicated by the displacement of the sheath from the referenced entry indicating mark 107 to any subsequent indicator 107.

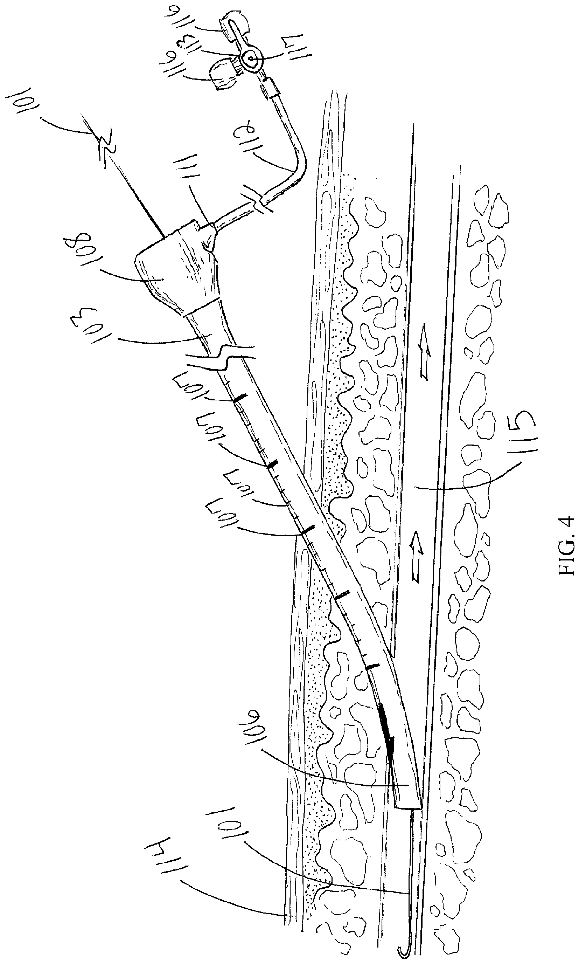

FIG. 4 is a side view of the sheath 103 shown in FIGS. 1-3 post withdrawal of the dilator component 102 from the sheath-dilator system 100, in accordance. After sheath 103 is correctly positioned within artery 115 (i.e. at the required depth for any subsequent procedures), the dilator may be disengaged from the sheath hub 108 and removed from the patient and sheath 103. As such, another device, such as a closure device, or another endovascular device, may be tracked down the sheath. The sheath hub 108 and body of the closure device are engageable in accordance with embodiments. In some embodiments, the sheath hub may connect to a closure device via a snap fit feature. In other embodiments, the sheath hub may include a slide and rotate channel for engaging the closure device. Such a channel may be the same channel used to engage the dilator hub. Once the sheath hub is engaged with a closure device the whole assembly may be retracted using the calibrated sheath depth indicators 107 to locate an implant deployable by the closure device against the inner wall of the arteriotomy. The closure device may then be cycled to secure the implant to the wall of the artery, thereby affecting an immediate seal. The closure device sheath assembly may then be removed from the tissue tract. A closure device as provided in any of US. Patent Application Nos. 11/413,636, filed April 28, 2006 and issued July 23, 2010 as US Pat. No. 7,753,935, 12/637,948, filed December 15, 2009, 12/749,729, filed March 30, 2010, 12/139,926, filed June 16, 2008, and 12/982,852, filed December 30, 2010, and U.S. Provisional Patent Application Serial No. 61/605,093, filed February 29, 2012, may be provided, as well as other closure devices or other medical devices.

FIG. 5 shows a schematic of a dilator hub with a blood signal port connected to a syringe in accordance with embodiments. Dilator 503, like dilator 102 in FIGS. 1-3 may be engageable with a sheath to form a sheath dilator system according to embodiments. Dilator 503 includes blood inlet holes 505, which provide a blood signal indicating entry into an artery. Dilator 503 also includes an opening 506 at the distal tip of the dilator 503. Opening 505 may be in fluid communication with opening 510 in hub 509 attached to the proximal end of dilator 503. Opening 510 may include a tube 501 that includes a connector 511, such as a luer connector. Connection 511 may releaseably connect to a device such as a syringe 502, which may be used to inject fluid 507 into an artery through opening 506 and/or 505 or may conversely be used to controllably extract blood from the artery. Additionally, the connector may be used to connect the dilator 503 with a syringe 502 containing saline, which may be used to flush the dilator lumen out and clear the dilator lumen(s) of any remaining air, and ensure that all ports are open. This avoids potential to introduce air emboli into the blood.

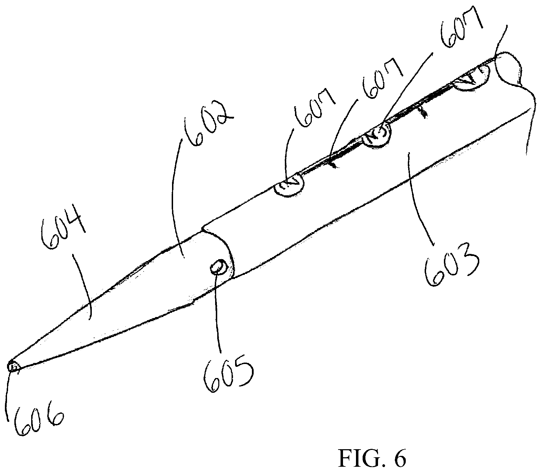

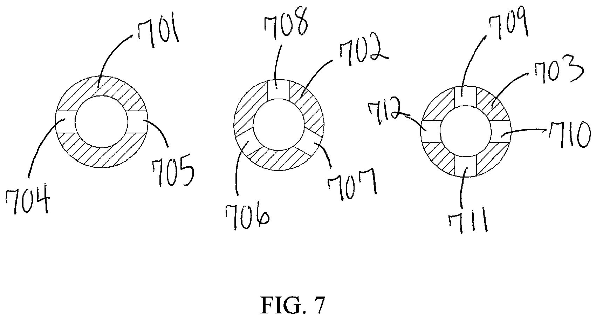

FIG. 6 is an isometric view of a sheath blood dilator in accordance with embodiments. The dilator includes an opening 606 for tracking the dilator down a guide wire. The dilator also includes a blood inlet hole 605 behind the tapered tip 604 of dilator 602 in accordance with embodiments. In this embodiment the blood inlet holes 605 are located on the O.D. of the dilator. The blood inlet hole in accordance with this embodiment is located immediately distal to the distal tip of sheath 603. These holes are in communication through the dilator tube, via at least a single lumen, or a multi lumen tube, with at least one hole the proximal hub of the dilator. The communication allows fluids, such as blood in the case of penetration into an artery through an arteriotomy, to flow. The holes when used in an endovascular procedure, will allow the user to know when the holes enter the artery, as the holes will provide a conduit for the arterial blood to flow through the dilator tube and exit the port on the proximal dilator hub. Given the relationship of the introducer sheath, which is operably connected to the dilator and hub, the user will have a means of knowing the location of the introducer sheath tip relative the arteriotomy as described above. Accordingly, indicators 607 may be appropriately referenced based on their location with respect to the user's skin. The inlet holes may be provided through a variety of methods according to examples, including punching the holes into a dilator extrusion during a secondary manufacturing operation. The dilator may include variants of the location, size and shape of the blood inlet holes in accordance with various embodiments, one of which is provided in FIG. 7.

FIG. 7 illustrates cross-sectional end views of various dilators having distinct blood inlet hole configurations in accordance with embodiments. Some embodiments incorporate one of the following: two holes 704 and 705 in a dilator 701 at 180 degrees to each other; three holes 706, 707, and 708 in dilator 702 at 120 degrees to each other; or four holes 709, 710, 711, and 712 in dilator 703 at 90 degrees to each other. The dilator may require more than one blood inlet hole to mitigate against the risk of this hole becoming inadvertently blocked e.g. manual digital pressure on the wound site might block or occlude a hole. With any of these single lumen options, the blood signalling lumen also accommodates the guide wire.

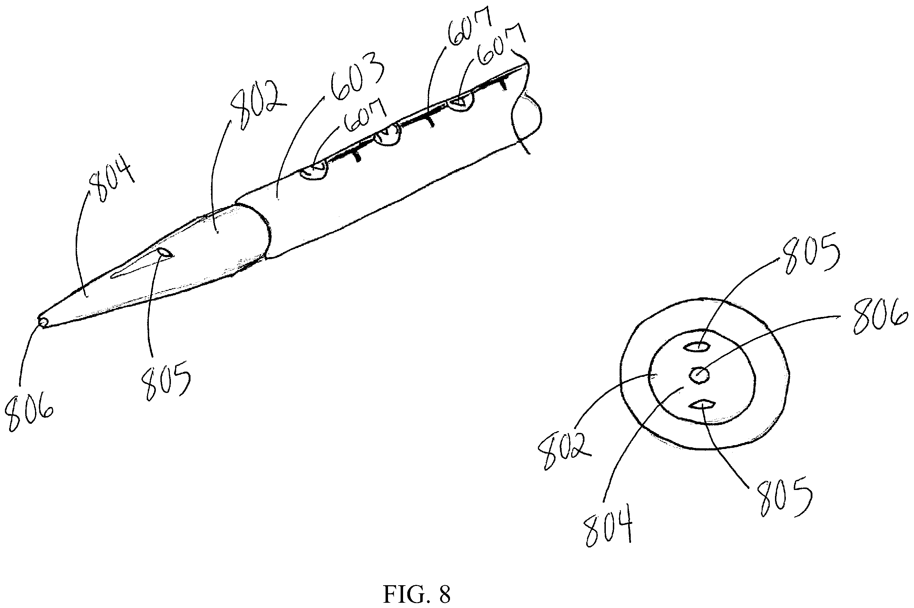

FIG. 8 is an isometric and front view of a sheath-dilator having an oval or elliptical blood inlet hole on the tapered section of the dilator portion in accordance with embodiments. In accordance with this embodiment, blood inlet holes 805 are located along the tapered section 804 of dilator 802. The dilator extends through sheath 603 having depth indicators 607 located thereon. Some benefits of such embodiments include the fact that the blood entry holes 805 are in line with the direction of the arterial blood flow, which may increase the potential for a stronger blood signal. Having these entry ports 805 elliptical (or other suitable shape) allows the opening to be kept low in profile, as shown in the front on view of Fig. 8, as compared to, e.g., a circular port. This is advantageous because it reduces the length of skive in front of the opening 805, shown in the isometric view of Fig. 8. Reducing this skive length reduces the potential of bleeding into the perivascular space during insertion. This bleeding occurs when the skive is both intra- and extra -arterial during insertion. Additionally, such an embodiment allows the blood signal lumens to be independent of the guide wire lumen 806. In accordance with some embodiment, a single lumen in the dilator tube may provide passageways to both the guide wire and fluid simultaneously. In such embodiments, the inlet holes may be formed, for example, as part of an RF tipping process for the forming the tapered tip. If the dilator is tracked into an artery which has an I.D. very close to or the same as the dilator O.D, then having the blood inlet lumen on the tapered section lowers the risk of the blood inlet hole being blocked or obstructed by the wall of the artery. Although the entry holes are oval or elliptical, it should be understood that other advantageous cross-sectional geometries, for example, crescent-shaped entry holes

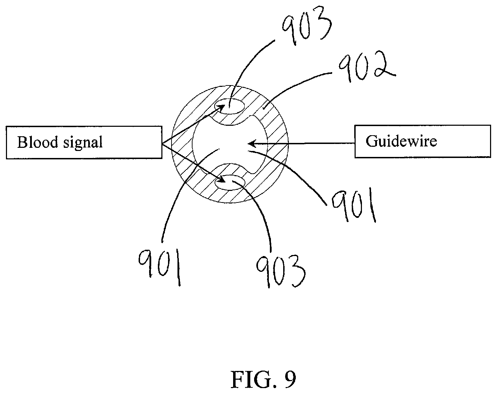

FIG. 9 is a cross section of a dilator with a multi-lumen configuration in accordance with embodiments. The dilator 902 according to this embodiment includes a lumen 901 for tracking dilator 902 down a guide wire. The dilator also includes two blood signal lumens 903 for transporting blood from an artery to a proximal end of the dilator. As demonstrated the lumen for any of such features, may have geometries other than purely circular geometries. Such lumen may have an oval/elliptical shape, a crescent-shape, or any other, e.g., irregular, shapes in accordance with various embodiments.

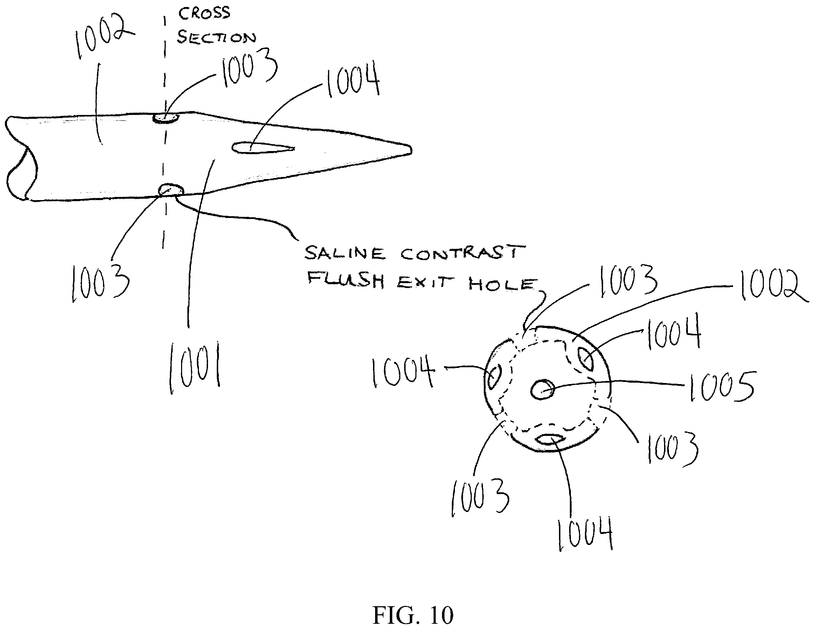

FIG. 10 is a side view and a cross-sectional view of a multi-lumen dilator having flushing holes in accordance with embodiments. The dilator 1001 includes a plurality of openings 1004 in the tapered section of the dilator. In addition, the multi lumen dilator may incorporate additional flushing holes 1003 in addition to the blood entry holes. These flushing holes may be used to flush saline or contrast media down through the dilator independent of the blood lumens. Accordingly, the holes may be used as flushing conduits for saline and contract and/ or as blood signal holes. Moreover, openings 1004 may be dedicated blood inlet/detection ports separate and independent of holes 1003 configured as flushing ports.

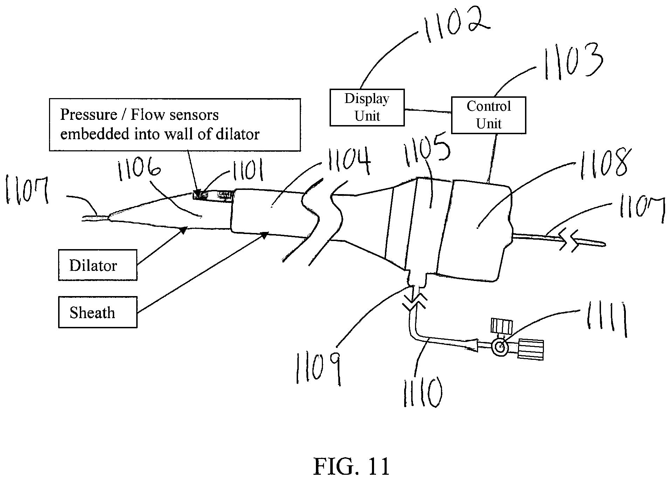

FIG. 11 illustrates a sheath-dilator having blood pressure sensors in accordance with embodiments. Another option for accurately positioning the sheath tip would be to incorporate an active blood detector such as one or more sensors 1101 into the wall of the dilator component. The sensors may include, but are not limited to, blood pressure sensors, tissue type sensors, and flow sensors in accordance with embodiments. The sensor may include other types of sensors capable of detecting blood flow. The sensor(s) 1101 may be connected to a control unit 1103 via a micro electrical wire or fiber-optic cable, which would travel back up through the I.D. of dilator 1106 and would interface with the control unit 1103 and a display unit 1102. Sensor(s) 1101 detect changes associated with the increase in pressure or flow due to penetration of the dilator tip into the lumen of the femoral artery as the sheath dilator system travels down guide wire 1107 into such an artery. As noted above, the dilator may incorporate one or more sensors and the sensors may be arranged in a linear arrangement extending longitudinally along the dilator or the sensors may be arranged in a configuration laterally traversing dilator 1106. In some embodiments, a distal sensor is positioned on the dilator to indicate when it enters into the blood stream and another sensor is positioned immediately adjacent to the distal tip of the sheath. When the sensor adjacent to the distal tip of the sheath enters the blood stream, the change detected by the sensor indicates to the operator that the sheath tip is positioned at the arteriotomy puncture site. Such an active system may be provided with embodiments of the sheath dilator system that also include engageable hubs 1105 and 1108, port 1109, tube 1110, and connector 1111, similar to those described with previous embodiments.

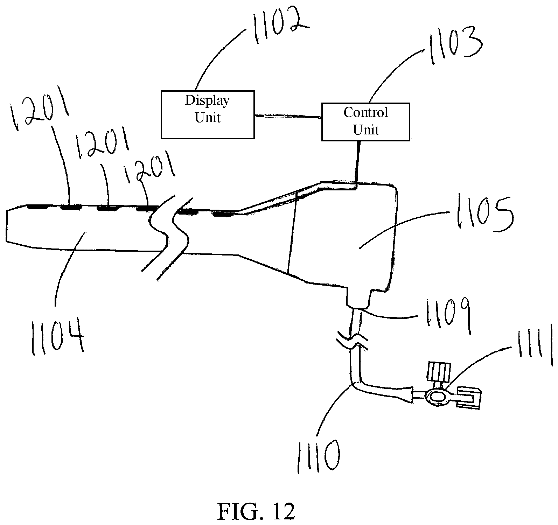

FIG. 12 shows a sheath assembly having sensors connected to a control unit and a display in accordance with embodiments. In this embodiment, the sheath 1104 includes sensors 1201 located thereon. Sensors 1201 provide alternative depth indicators to the passive graduation marks demonstrated in earlier embodiments. The sensors may be connected to control unit 1103 and display unit 1102 via a micro electrical wire or a fiber optic cable and may operate in concert with the sensors located on the dilator, such sensors 1201 are automatically activated by the detection of blood flow via the sensors on the sheath. Sensors 1201 may also be calibrated with respect to one another such that the distance between the sensors is known and such that the changes associated with penetrating the skin of the patient, such as pressure changes may be referenced with respect to other sensors to indicate the depth of penetration since entry into an artery via an arteriotomy by the sheath. Such sensors may continue to provide output after the dilator is removed and a device, such as a closure device is attached to the sheath. When a closure device is attached to the sheath sensors 1201 may provide feedback in connection with positioning the closure device accurate with respect to the arteriotomy of the vessel in order to deploy the closure device at the optimal location.

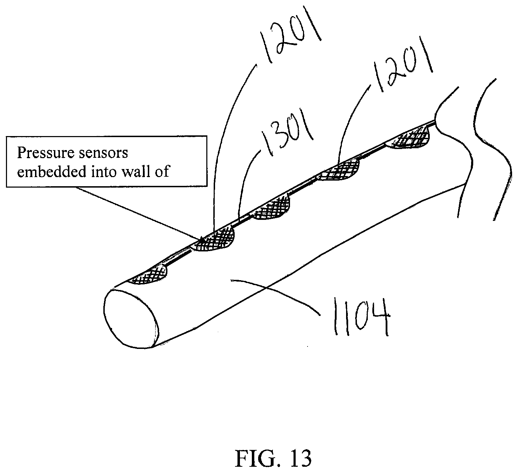

FIG. 13 shows an isometric view of a sheath having embedded sensors as demonstrated in FIG. 12 in accordance with embodiments of the present invention. The sensors may be provided in an integral fashion with respect to sheath 1104 such that they maintain the normal outer diameter of the sheath. Sensors 1201 may be electrically connected to one another via connection 1301. As noted above, the sensors may include, but are not limited to, pressure sensors and flow sensors. The sensors may be embedded into the wall of the sheath during the extrusion process. The sensors may be provided as an alternative to or in conjunction with the graduation marks disclosed in various embodiments of the invention.

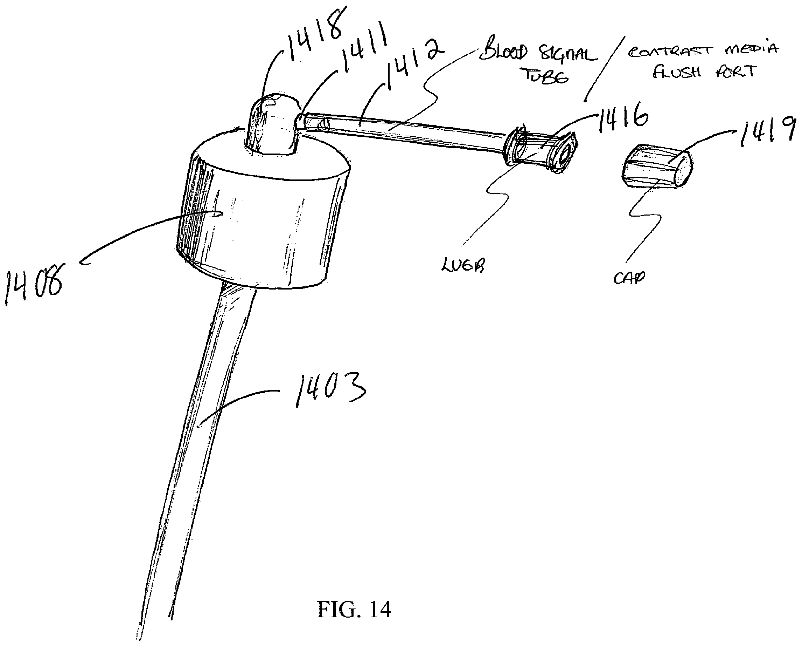

FIG 14 illustrates a hub for a dilator in accordance with embodiments. Extension 1403 is representative of a dilator used in accordance with embodiments. Hub 1408 is connected to at least one of the dilator 1403 extending therefrom. Hub 1408 facilitates fluid communication between the extension 1403 and any fluid source or reservoir (not shown) maintained at the luer connection site 1416. In the absence of such a source or reservoir, connection site 1416 may be capped by cap 1419 to prevent contamination. In the presence of such a source or reservoir, fluid may be added or removed from the dilator or sheath by traveling from the connection site 1416, down tube 1412, into port 1411, and through elbow 1418 at the top of hub 1408. The hub, port, tube, and connection site may all be composed of plastic in accordance with various embodiments. The hub, elbow, and port may be manufactured using a molding process such as an injection molding process.

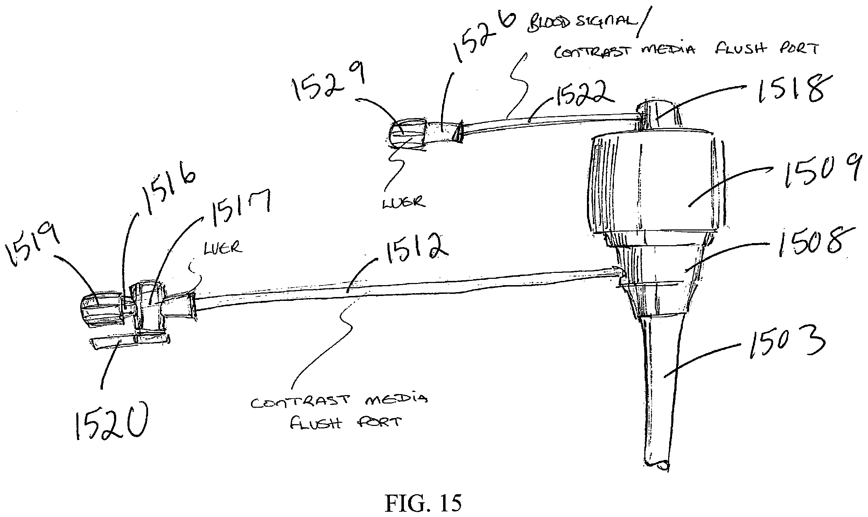

FIG 15 illustrates another hub for a sheath-dilator system in accordance with embodiments. The hub illustrated in FIG. 15 is a multi-part hub with multiple input/output fluid channels. In accordance with embodiments a lower hub portion 1508 may be adjacent to sheath 1503. Hub 1508 may provide a fluid communication path between a source or reservoir (not shown) via tube 1512 and connector 1517. Connector 1517 may be a multi-way connector such that 2, 3, or more sources or reservoirs may be brought into fluid communications with sheath 1503. The depicted connector 1517 allows two sources to be connected at sites 1516, one of which is capped via cap 1519 in the illustrated embodiment. Connector 1517 also includes a valve controlled by valve stem 1520, the rotary motion of which allows or prevents any source or reservoir maintained at either of the connections sites from being in fluid communication with tube 1512 and hence hub 1508 and sheath 1503. Upper hub portion 1509 may provide a distinct fluid communication path via elbow 1518 and tube 1522. As demonstrated in FIG. 15, tube 1522 (and similarly tube 1512) may include a single-way connector such as connector 1526. The connector may include a cap 1529 when no source or reservoir is present. The connector 1526 may be a luer type connector. Each of the connectors 1517 and 1526 and their respective tubes 1512 an 1522 may allow outward bound fluid communication (i.e. blood or other bodily fluids flowing from sheath 1503 to the connector to achieve a blood signal indicator) or inward bound communication (i.e. fluid such as a contrast media or saline flowing from the connector towards the sheath) in accordance with embodiments. The upper hub 1509 may provide fluid communication between a reservoir and the dilator, while the lower hub 1508 may provide fluid communication between a source and the sheath or vice versa in accordance with embodiments. Accordingly, one hub may be used solely for outbound flow, particularly flow associated with indicating entry into a vessel by outward flow of blood through the dilator, while the other hub may be used solely for inbound flow of contrast media, saline solution, or other fluids being introduced into a patient. Alternatively, in some embodiments, both hubs may facilitate bi-direction fluid communication. The hubs may also be engaged such that connection tubes 1512 and 1522 extend in the same direction or in distinct directions in accordance with various embodiments. Hubs 1508 and 1509 may be rotatably connected in accordance with embodiments to facilitate positioning the respective connection tubes as desired.

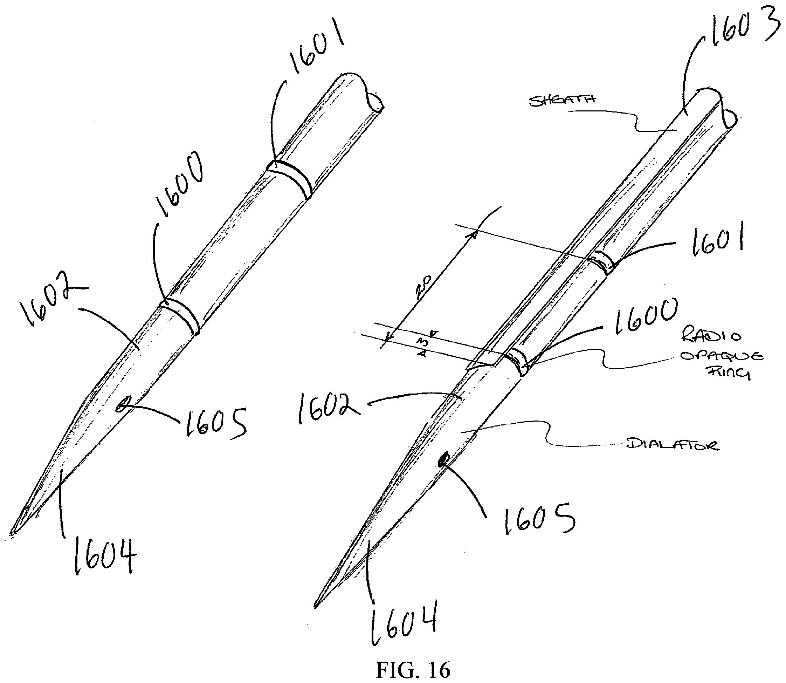

FIG. 16 shows a sheath dilator system that includes radiopaque markers in accordance with embodiments. Dilator 1602 may include one or more radiopaque markers in accordance with embodiments. The markers may be positioned at specified distances from the tapered tip 1604 of the dilator or from specified distances from the blood inlet hole(s) 1605 on the tapered tip of the dilator. The markers 1600 and 1601 may be positioned concentrically on dilator 1602 and within sheath 1603. The markers may be flush with the dilator in accordance with embodiments. The markers function is to provide visual indicators to an operator of the location of the dilator and the sheath. The markers, which may have a width of 3mm, may provide distinct location identifiers. For example, the first marker 1600 may be positioned adjacent to or just short of the distal tip of sheath 1603 and accordingly, provides a visual indicator of the location of the distal tip of the sheath to an operator. The second marker 1601 may indicate the position of the dilator relative to the sheath distal tip. Once the proximal radiopaque marker 1601 is positioned within the arteriotomy, an operator may be apprised of the distal tip of the sheath being positioned in the artery lumen and a closure device may subsequently be introduced into the artery lumen based on this position indicative information. Marker bands may be manufactured from a platinum/iridium metal marker band which is swaged onto the circumference of the dilator.

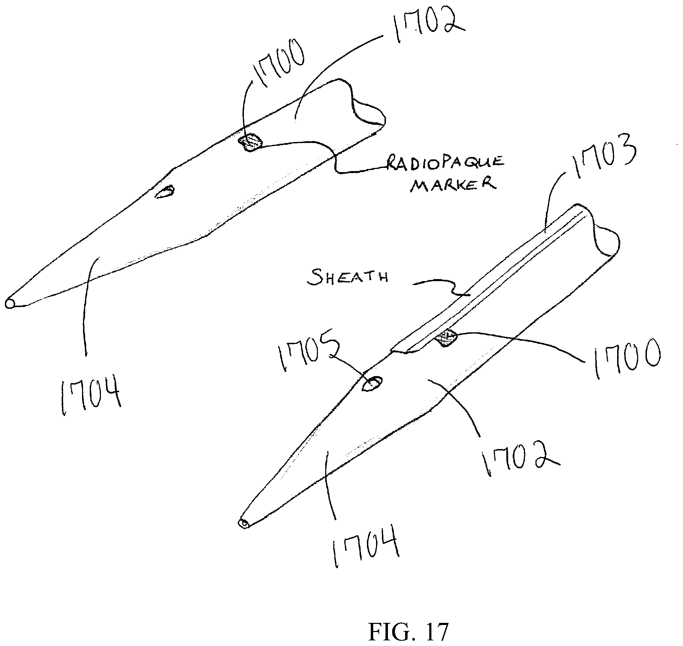

FIG. 17 shows another sheath dilator system that includes radiopaque markers in accordance with embodiments. Dilator 1702 may include one or more radiopaque markers 1700 in accordance with embodiments. The markers may have a variety of geometric shapes, such as a square, circle, or other shapes forming a point in contrast to the band markers shown in FIG. 16. The markers may be positioned at specified distances from the tapered tip 1704 of the dilator or from specified distances from the blood inlet hole(s) 1705 on the tapered tip of the dilator. The markers may be flush with the dilator in accordance with embodiments. The markers function is to provide visual indicators to an operator of the location of the dilator and the sheath, similar to the function of markers 1600 and 1601 of FIG. 16. Marker 1700 may also be manufactured from a platinum/iridium metal marker band which is swaged onto the circumference of the dilator.

## Claims

1. A medical device comprising:
a sheath (103, 603, 1104) having a proximal end and a distal end, the sheath (103, 603) having an opening at both the proximal end and the distal end; and
a removable dilator (102, 503, 602, 701, 702, 703, 802, 902, 1001, 1602) having a distal end and a proximal end, the dilator having at least one blood inlet opening (105, 505, 605, 704, 705, 706, 707, 708, 709, 710, 711, 712, 805, 903, 1004, 1605, 1705) disposed on a distal end of the dilator, the blood inlet opening being in fluid communication with a blood outlet (110, 510) at the proximal end of the dilator, the dilator positionable within the sheath such that a tapered portion (104, 504, 604, 804, 1001) of the distal end of the dilator extends through the opening in the distal end of the sheath, the proximal end of the dilator extends through the opening in the proximal end of the sheath, and at least a portion of the at least one blood inlet opening is adjacent to the distal end of the sheath,
wherein the dilator blood inlet opening (105, 505, 605, 704, 705, 706, 707, 708, 709, 710, 711, 712, 805, 903, 1004, 1605, 1705) is disposed on the tapered portion, **characterised in that** the blood inlet opening (105, 505, 605, 704, 705, 706, 707, 708, 709, 710, 711, 712, 805, 903, 1004, 1605, 1705) has a crescent-shaped cross-section when viewed along a longitudinal axis of the dilator.

2. A medical device according to claim 1, wherein the sheath further comprises one or more depth indicators (107, 607, 1201, 1600, 1601, 1700) affixed thereto.

3. A medical device according to claim 1, wherein the sheath further comprises a hub (108) at the proximal end of the sheath and the dilator comprises a hub (109) at the proximal end of the dilator, the sheath hub (108) releasably engaged with the dilator hub (109).

4. A medical device according to claim 3, wherein the blood outlet (110, 510) is in the dilator hub (109).

5. A medical device according to claim 1, wherein the dilator further comprises at least one opening (1003, 1004) at the distal tapered end, the at least one opening (1003, 1004) in fluid communication with one or more lumens within the dilator, the one or more lumens in fluid communication with a port (1110) in the dilator hub, whereby liquid is transferable between the opening in the distal tapered end of the dilator and the port in the dilator hub.

6. A medical device according to claim 2, wherein the depth indicators include graduation marks.

7. A medical device according to claim 5, further comprising a luer connector (113) extending from the port (111) in the dilator hub, the luer connector creating a fluid pathway between the port and at least one of a 2 and 3 way tap (117).

8. A medical device according to claim 1, wherein the dilator includes an internal lumen (606, 806) extending the entire length of the dilator and adapted for tracking over a guide wire (101).

9. A medical device according to claim 1, wherein the transition between the distal end of the sheath and the dilator is substantially smooth.

10. A medical device according to claim 3, wherein the sheath hub includes an interface connectable to an endovascular device.

11. A medical device according to claim 10 wherein the endovascular device is a closure device.

12. A medical device according to claim 1, further comprising at least one sensor (1101, 1201) on the outer surface of the sheath.

13. A medical device according to claim 1, further comprising a linear array of sensors (1101, 1201) located along the outer surface of the sheath.

14. A medical device according to claim 13, wherein the linear array of sensors is an array of tissue type sensors.

15. A medical device according to claim 12, wherein the at least one sensor is a pressure sensor.

16. A medical device according to claim 1, further comprising one or more radiopaque markers (1600, 1601, 1700) positioned on the dilator.

## Patentansprüche

1. Medizinische Vorrichtung, die Folgendes umfasst:
ein Hüllrohr (103, 603, 1104) mit einem proximalen Ende und einem distalen Ende, wobei das Hüllrohr (103, 603) an sowohl dem proximalen Ende als auch dem distalen Ende eine Öffnung aufweist; und
einen abnehmbaren Dilatator (102, 503, 602, 701, 702, 703, 802, 902, 1001, 1602) mit einem distalen Ende und einem proximalen Ende, wobei der Dilatator mindestens eine an einem distalen Ende des Dilatators angeordnete Bluteinlassöffnung (105, 505, 605, 704, 705, 706, 707, 708, 709, 710, 711, 712, 805, 903, 1004, 1605, 1705) aufweist, wobei sich die Bluteinlassöffnung mit einem Blutauslass (110, 510) an dem proximalen Ende des Dilatators in Fluidverbindung befindet, wobei der Dilatator derart innerhalb des Hüllrohrs positionierbar ist, dass sich ein verjüngter Abschnitt (104, 504, 604, 804, 1001) des distalen Endes des Dilatators durch die Öffnung in dem distalen Ende des Hüllrohrs hindurch erstreckt, sich das proximale Ende des Dilatators durch die Öffnung in dem proximalen Ende des Hüllrohrs erstreckt und mindestens ein Abschnitt der mindestens einen Bluteinlassöffnung dem distalen Ende des Hüllrohrs benachbart ist,
wobei die Dilatatorbluteinlassöffnung (105, 505, 605, 704, 705, 706, 707, 708, 709, 710, 711, 712, 805, 903, 1004, 1605, 1705) an dem verjüngten Abschnitt angeordnet ist, **dadurch gekennzeichnet, dass** die Bluteinlassöffnung (105, 505, 605, 704, 705, 706, 707, 708, 709, 710, 711, 712, 805, 903, 1004, 1605, 1705) bei Betrachtung entlang einer Längsachse des Dilatators einen sichelförmigen Querschnitt aufweist.

2. Medizinische Vorrichtung nach Anspruch 1, wobei das Hüllrohr weiter einen oder mehrere daran angebrachte Tiefenanzeiger (107, 607, 1201, 1600, 1601, 1700) umfasst.

3. Medizinische Vorrichtung nach Anspruch 1, wobei das Hüllrohr weiter einen Ansatz (108) an dem proximalen Ende des Hüllrohrs umfasst und der Dilatator einen Ansatz (109) an dem proximalen Ende des Dilatators umfasst, wobei sich der Hüllrohransatz (108) mit dem Dilatatoransatz (109) lösbar im Eingriff befindet.

4. Medizinische Vorrichtung nach Anspruch 3, wobei sich der Blutauslass (110, 510) in dem Dilatatoransatz (109) befindet.

5. Medizinische Vorrichtung nach Anspruch 1, wobei der Dilatator weiter mindestens eine Öffnung (1003, 1004) an dem distalen verjüngten Ende umfasst, wobei sich die mindestens eine Öffnung (1003, 1004) mit einem oder mehreren Lumen in dem Dilatator in Fluidverbindung befindet, wobei sich das eine oder die mehreren Lumen mit einem Anschluss (1110) in dem Dilatatoransatz in Fluidverbindung befinden, wodurch Flüssigkeit zwischen der Öffnung in dem distalen verjüngten Ende des Dilatators und dem Anschluss in dem Dilatatoransatz überführbar ist.

6. Medizinische Vorrichtung nach Anspruch 2, wobei die Tiefenanzeiger Teilstriche umfassen.

7. Medizinische Vorrichtung nach Anspruch 5, weiter umfassend einen Luerverbinder (113), der sich von dem Anschluss (111) in dem Dilatatoransatz erstreckt, wobei der Luerverbinder einen Fluidpfad zwischen dem Anschluss und einem 2- und/oder einem 3-Wege-Hahn (117) erzeugt.

8. Medizinische Vorrichtung nach Anspruch 1, wobei der Dilatator ein inneres Lumen (606, 806) umfasst, dass sich über die gesamte Länge des Dilatators erstreckt und zum Laufen über einen Führungsdraht (101) angepasst ist.

9. Medizinische Vorrichtung nach Anspruch 1, wobei der Übergang zwischen dem distalen Ende des Hüllrohrs und dem Dilatator im Wesentlichen glatt ist.

10. Medizinische Vorrichtung nach Anspruch 3, wobei der Hüllrohransatz eine Schnittstelle umfasst, die mit einer endovaskulären Vorrichtung verbindbar ist.

11. Medizinische Vorrichtung nach Anspruch 10, wobei es sich bei der endovaskulären Vorrichtung um eine Verschlussvorrichtung handelt.

12. Medizinische Vorrichtung nach Anspruch 1, weiter umfassend mindestens einen Sensor (1101, 1201) an der äußeren Oberfläche des Hüllrohrs.

13. Medizinische Vorrichtung nach Anspruch 1, weiter umfassend eine lineare Anordnung von Sensoren (1101, 1201), die sich entlang der äußeren Oberfläche des Hüllrohrs befinden.

14. Medizinische Vorrichtung nach Anspruch 13, wobei es sich bei der linearen Anordnung von Sensoren um eine Anordnung von Gewebesensoren handelt.

15. Medizinische Vorrichtung nach Anspruch 12, wobei es sich bei dem mindestens einen Sensor um einen Drucksensor handelt.

16. Medizinische Vorrichtung nach Anspruch 1, weiter umfassend eine oder mehrere an dem Dilatator positionierte röntgendichte Markierungen (1600, 1601, 1700).

## Revendications

1. Dispositif médical comprenant :
une gaine (103, 603, 1104) ayant une extrémité proximale et une extrémité distale, la gaine (103, 603) ayant une ouverture à la fois au niveau de l'extrémité proximale et de l'extrémité distale ; et
un dilatateur amovible (102, 503, 602, 701, 702, 703, 802, 902, 1001, 1602) ayant une extrémité distale et une extrémité proximale, le dilatateur ayant au moins une ouverture d'entrée de sang (105, 505, 605, 704, 705, 706, 707, 708, 709, 710, 711, 712, 805, 903, 1004, 1605, 1705) disposée sur une extrémité distale du dilatateur, l'ouverture d'entrée de sang étant en communication fluidique avec une sortie de sang (110, 510) au niveau de l'extrémité proximale du dilatateur, le dilatateur pouvant être positionné au sein de la gaine de sorte qu'une partie conique (104, 504, 604, 804, 1001) de l'extrémité distale du dilatateur s'étend à travers l'ouverture dans l'extrémité distale de la gaine, l'extrémité proximale du dilatateur s'étend à travers l'ouverture dans l'extrémité proximale de la gaine, et au moins une partie de l'au moins une ouverture d'entrée de sang est adjacente à l'extrémité distale de la gaine,
dans lequel l'ouverture d'entrée de sang du dilatateur (105, 505, 605, 704, 705, 706, 707, 708, 709, 710, 711, 712, 805, 903, 1004, 1605, 1705) est disposée sur la partie conique, **caractérisé en ce que** l'ouverture d'entrée de sang (105, 505, 605, 704, 705, 706, 707, 708, 709, 710, 711, 712, 805, 903, 1004, 1605, 1705) aune section transversale en forme de croissant lorsqu'elle est observée le long de l'axe longitudinal du dilatateur.

2. Dispositif médical selon la revendication 1, dans lequel la gaine comprend en outre un ou plusieurs indicateurs de profondeur (107, 607, 1201, 1600, 1601, 1700) fixés sur celle-ci.

3. Dispositif médical selon la revendication 1, dans lequel la gaine comprend en outre un collet (108) au niveau de l'extrémité proximale de la gaine et le dilatateur comprend un collet (109) au niveau de l'extrémité proximale du dilatateur, le collet de gaine (108) étant en prise de manière libérable avec le collet de dilatateur (109).

4. Dispositif médical selon la revendication 3, dans lequel la sortie de sang (110, 510) se trouve dans le collet de dilatateur (109).

5. Dispositif médical selon la revendication 1, dans lequel le dilatateur comprend en outre au moins une ouverture (1003, 1004) au niveau de l'extrémité conique distale, l'au moins une ouverture (1003, 1004) étant en communication fluidique avec une ou plusieurs lumières au sein du dilatateur, les une ou plusieurs lumières étant en communication fluidique avec un orifice (1110) dans le collet de dilatateur, ce par quoi du liquide peut être transféré entre l'ouverture dans l'extrémité conique distale du dilatateur et l'orifice dans le collet de dilatateur.

6. Dispositif médical selon la revendication 2, dans lequel les indicateurs de profondeur incluent des marques de graduation.

7. Dispositif médical selon la revendication 5, comprenant en outre un connecteur luer (113) qui s'étend à partir de l'orifice (111) dans le collet de dilatateur, le connecteur luer créant un trajet de fluide entre l'orifice et au moins l'un d'un robinet à 2 et à 3 voies (117).

8. Dispositif médical selon la revendication 1, dans lequel le dilatateur inclut une lumière interne (606, 806) qui s'étend sur toute la longueur du dilatateur et adaptée pour le suivi d'un fil guide (101).

9. Dispositif médical selon la revendication 1, dans lequel la transition entre l'extrémité distale de la gaine et le dilatateur est sensiblement lisse.

10. Dispositif médical selon la revendication 3, dans lequel le collet de gaine inclut une interface pouvant être connectée à un dispositif endovasculaire.

11. Dispositif médical selon la revendication 10, dans lequel le dispositif endovasculaire est un dispositif de fermeture.

12. Dispositif médical selon la revendication 1, comprenant en outre au moins un capteur (1101, 1201) sur la surface externe de la gaine.

13. Dispositif médical selon la revendication 1, comprenant en outre un réseau linéaire de capteurs (1101, 1201) situés le long de la surface externe de la gaine.

14. Dispositif médical selon la revendication 13, dans lequel le réseau linéaire de capteurs est un réseau de capteurs de type de tissu.

15. Dispositif médical selon la revendication 12, dans lequel l'au moins un capteur est un capteur de pression.

16. Dispositif médical selon la revendication 1, comprenant en outre un ou plusieurs marqueurs radio-opaques (1600, 1601, 1700) positionnés sur le dilatateur.
